# EUROPÄISCHE PATENTANMELDUNG

(11) **EP 3 443 887 A1**
(43) Veröffentlichungstag der Anmeldung: **20.02.2019**
(21) Anmeldenummer: 17186913.4
(22) Anmeldetag: 18.08.2017
(51) Int. Cl.: A61B 5/00, A61B 5/145, A61B 5/1455

(54) **VORRICHTUNG UND VERFAHREN ZUR NICHT-INVASIVEN BESTIMMUNG VON ANALYTEN OHNE EXTERNE STRAHLUNGSQUELLE**

(71) Anmelder: Swiss Spectral AG, 8400 Winterthur (CH)
(72) Erfinder: Die Erfindernennung liegt noch nicht vor
(74) Vertreter: Weickmann & Weickmann PartmbB

(57) **Zusammenfassung**

Die vorliegende Erfindung betrifft eine Vorrichtung und ein Verfahren zur nicht-invasiven quantitativen Bestimmung eines Analyten in Blut, insbesondere zur nicht-invasiven quantitativen Bestimmung von Glucose in Kapillarblut.

## Beschreibung

Die vorliegende Erfindung betrifft eine Vorrichtung und ein Verfahren zur nicht-invasiven quantitativen Bestimmung eines Analyten in Blut, insbesondere zur nicht-invasiven quantitativen Bestimmung von Glucose in Kapillarblut.

Im Jahr 2016 leiden etwa 415 Millionen Menschen an Diabetes. Für das Jahr 2040 ist ein Anstieg auf über 640 Millionen Menschen zu erwarten. Für Diabetes-Patienten ist eine genaue Überwachung der Konzentration von Glucose im Blut erforderlich, um eine entsprechende Medikation zu ermöglichen. Es besteht daher ein hoher Bedarf an zuverlässigen und für den Patienten einfach anwendbaren Methoden zur Bestimmung von Glucose in Blut.

Derzeit basiert die Bestimmung von Glucose in Blut überwiegend auf invasiven Methoden. Hierbei wird dem betreffenden Patienten entweder eine Blutprobe entnommen und anschließend einem *in vitro-*Test unterzogen oder es wird ein Sensor implantiert, der zur Bestimmung von Glucose *in vivo* dient. Ein Nachteil solcher invasiven Methoden ist, dass sie für den Patienten schmerzhaft und unbequem sind.

Zur Vermeidung dieses Nachteils wurden bereits zahlreiche Ansätze zur nicht-invasiven Bestimmung der Konzentration von Glucose in Blut entwickelt. Keiner dieser Ansätze hat jedoch bisher kommerzielle Bedeutung erlangt.

WO 2014/0206549 beschreibt eine Vorrichtung zum Messen von Rohdaten zur nicht-invasiven Bestimmung eines Blutparameters, beispielsweise der Konzentration von Glucose, wobei Infrarot (IR)-Strahlung aus einer externen Strahlungsquelle flächig an mehreren Messstellen in die zu untersuchende Körperoberfläche des Patienten eingekoppelt wird und die in der Körperoberfläche erzeugte IR-Strahlung an mehreren Messstellen von einer Sensoreinrichtung erfasst wird. Nachteilig an diesem Verfahren ist jedoch, dass es einen hohen apparativen Aufwand erfordert.

Durch die vorliegende Erfindung werden eine Vorrichtung und ein Verfahren zur nicht-invasiven quantitativen Bestimmung eines Analyten in einer Körperflüssigkeit bereitgestellt, mit denen die Nachteile des Standes der Technik vermieden werden können.

Die Erfindung beruht auf der Erkenntnis, dass eine einfache, schnelle und ausreichend genaue quantitative Bestimmung eines Analyten im Blut eines Testsubjekts durch nicht-invasive Methoden möglich ist. Dabei wird aus einem vorbestimmten Körperbereich des Testsubjekts stammende körpereigene IR-Strahlung bei mindestens zwei unterschiedlichen Wellenlängen oder Wellenlängenbereichen in der Region von 0,7-20 µm, vorzugsweise von 3-20 µm, besonders bevorzugt von 8-12 µm, separat erfasst. Hierzu erfolgt die Erfassung bei einer ersten Wellenlänge oder einem ersten Wellenlängenbereich, wo die Intensität der reflektierten IR-Strahlung von der Konzentration des zu bestimmenden Analyten im Wesentlichen unabhängig ist, und unabhängig davon bei einer zweiten Wellenlänge oder einem zweiten Wellenlängenbereich, wo sich die Intensität der reflektierten IR-Strahlung abhängig von der Konzentration des zu bestimmenden Analyten ändert. Durch differenzielle Auswertung der Signale von beiden Wellenlängen bzw. Wellenlängenbereichen kann die Konzentration des Analyten bestimmt werden.

Überraschenderweise wurde festgestellt, dass eine quantitative nicht-invasive Bestimmung von Analyten in Blut durch Auswertung der vom Körper emittierten IR-Strahlung ohne Verwendung externer Strahlungsquellen möglich ist. Die durch die Körpertemperatur des zu untersuchenden Körperbereichs, die üblicherweise im Bereich von 30°C bis 38°C liegt, emittierte IR-Strahlung kann die von einer externen IR-Strahlungsquelle emittierte und vom Körper reflektierte Strahlung ersetzen. Folglich betrifft die vorliegende Erfindung eine Vorrichtung und eine Verfahren zur nicht-invasiven quantitativen Bestimmung eines Analyten, insbesondere von Glucose, durch Auswertung der körpereigenen IR-Strahlung.

Eine Bestimmung von Glucose durch Messung von aus einer externen Strahlungsquelle stammenden IR-Strahlung bei zwei unterschiedlichen Wellenlängen wird bereits in WO 81/00622 vorgeschlagen, wobei auf die Offenbarung dieses Dokuments ausdrücklich Bezug genommen wird. Allerdings ermöglicht diese im Stand der Technik beschriebene Methode keine ausreichend genaue, nicht-invasive Bestimmung der Konzentration eines Analyten im Blut eines Testsubjekts.

Eine Ursache hierfür ist, dass der zu bestimmende Analyt möglicherweise nicht nur Blut, sondern auch im epidermalen und/oder dermalen Gewebe vorliegt, wobei sich die Gewebekonzentration beispielsweise bei Glucose deutlich von der Blutkonzentration unterscheiden kann. Somit ist die aus dem untersuchten Körperbereich stammende IR-Strahlung nicht notwendigerweise spezifisch für die Konzentration des Analyten in Blut. Dies kann zu einer unrichtigen Interpretation des Messergebnisses führen.

Um Nachteile der in WO 81/00622 beschriebenen Messmethode zu vermeiden, erfolgt gemäß vorliegender Erfindung eine selektive Auswertung von körpereigener IR-Strahlung in der Region von 0,7-20 µm, vorzugsweise von 3-20 µm, besonders bevorzugt von 8-12 µm, aus oberflächennahen Blutgefäßen des untersuchten Körperbereichs, insbesondere eine selektive Auswertung von körpereigener IR-Strahlung aus den Kapillarblutgefäßen der Dermis. Die Erfinder haben nun festgestellt, dass körpereigene IR-Strahlung aus mehreren Komponenten zusammengesetzt ist, die separat analysiert werden können. Die aus oberflächennahen Blutgefäßen stammende Komponente der körpereigenen IR-Strahlung weist eine von der arteriellen Pulsfrequenz des Testsubjekts abhängige zeitliche Variation auf. Aufgrund dieser Variation kann eine Diskriminierung zwischen sich mit der Pulsfrequenz ändernden Signalen und von der Pulsfrequenz unabhängigen Signalen im Rahmen der Auswertung vorgenommen werden. Auf diese Weise kann überraschenderweise die Konzentration des Analyten im Blut des Testsubjekts, beispielsweise die Konzentration von Glucose, mit hoher Genauigkeit bestimmt werden.

Ein erster Aspekt der vorliegenden Erfindung betrifft somit eine Vorrichtung zur nicht-invasiven quantitativen Bestimmung eines Analyten im Blut eines Testsubjekts, umfassend:
(a) eine Einheit zur Aufnahme eines von dem Testsubjekt stammenden Körperbereichs,
(b) eine Einheit zur Erfassung von körpereigener IR-Strahlung aus dem Körperbereich, die zur separaten Erfassung von IR-Strahlung bei mindestens zwei unterschiedlichen Wellenlängen oder Wellenlängenbereichen in der Region von 0,7-20 µm, vorzugsweise von 3-20 µm, besonders bevorzugt von 8-12 µm, eingerichtet ist, wobei bei einer ersten Wellenlänge oder einem ersten Wellenlängenbereich die Intensität der körpereigenen IR-Strahlung von der Konzentration des zu bestimmenden Analyten im Wesentlichen unabhängig ist, und
   wobei bei einer zweiten Wellenlänge oder einem zweiten Wellenlängenbereich sich die Intensität der körpereigenen IR-Strahlung abhängig von der Konzentration des zu bestimmenden Analyten ändert und
   wobei die Einheit gegebenenfalls zusätzlich zur unspezifischen Erfassung von körpereigener IR-Strahlung eingerichtet ist, und
(c) eine Einheit, die zur Auswertung der von der Erfassungseinheit (c) stammenden Signale und zur Ermittlung der Konzentration des Analyten auf Basis der ausgewerteten Signale eingerichtet ist,
   wobei die Einheit zur selektiven Auswertung von körpereigener IR-Strahlung eingerichtet ist, die aus Blutgefäßen des bestrahlten Körperbereichs, insbesondere aus Kapillarblutgefäßen der Dermis stammt.

Die erfindungsgemäße Vorrichtung enthält nicht notwendigerweise eine externe IR-Strahlungsquelle, da sie zur Erfassung und Auswertung von körpereigener IR-Strahlung eingerichtet ist. Diese körpereigene IR-Strahlung wird durch den in die Aufnahmeeinheit (a) eingebrachten zu untersuchenden Körperbereich emittiert. Schwankungen in der Körpertemperatur von ± 5 C° sind nicht relevant, da eine separate Erfassung von IR-Strahlung bei mindestens zwei unterschiedlichen Wellenlängen oder Wellenlängenbereichen erfolgt. Gegebenenfalls können Schwankungen der Körpertemperatur durch einen gegebenenfalls vorhandenen Temperatursensor kompensiert werden.

Die erfindungsgemäße Vorrichtung ist zur Aufnahme eines Körperbereichs eines Testsubjekts, insbesondere zur Aufnahme eines Körperbereichs eines menschlichen Testsubjekts, z.B. einer Fingerkuppe, eines Ohrläppchens oder einer Ferse oder Teilen davon, vorgesehen, um die davon emittierte körpereigene IR-Strahlung zu bestimmen. Hierzu enthält die Vorrichtung eine Einheit zur Aufnahme des zu bestrahlenden Körperbereichs, die beispielsweise ein Auflageelement für den betreffenden Körperbereich, z.B. die Fingerkuppe, umfassen kann. Die Form des Auflageelements ist dem Körperbereich angepasst, an dem die Bestimmung durchgeführt wird. So kann etwa ein planares Auflageelement vorgesehen sein.

Günstigerweise ist das Auflageelement zumindest teilweise transparent für IR-Strahlung in der Region von 0,7-20 µm, vorzugsweise von 3-20 µm, besonders bevorzugt von 8-12 µm, um ein ungehindertes Durchdringen der von dem zu untersuchenden Körperbereich emittierten IR-Strahlung im Bereich der ersten und zweiten Messwellenlänge zu ermöglichen. Beispiele für geeignete Materialien sind Silicium, Germanium oder organische IR-transparente Polymere. Das Auflageelement kann in jeder geeigneten Form ausgebildet sein, beispielsweise als Scheibe. Des Weiteren hat es sich als günstig herausgestellt, dass das Auflageelement mit einem Temperaturstabilisierungselement, insbesondere mit einem Peltier-Element versehen ist, um ein unerwünschtes Aufheizen des untersuchenden Körperbereichs zu vermeiden. Beispielsweise kann das Temperaturstabilisierungselement so eingerichtet sein, dass es eine Temperatur des Auflageelements von 31 °C oder weniger, z.B. von etwa 1-31 °C und bevorzugt von etwa 25-30 °C einstellen kann.

Vorzugsweise umfasst das Auflageelement Mittel, z.B. Sensoren, zur Erfassung und/oder Kontrolle der Auflageposition und/oder des Auflagedrucks für den zu untersuchenden Körperbereich. Dabei können die Auflageposition und/oder der Auflagedruck individuell für jeden Körperbereich erfasst und gegebenenfalls adaptiert werden. Die individuelle Adaption kann z.B. einen ein- oder mehrmaligen Abgleich des von der erfindungsgemäßen nicht-invasiven Vorrichtung stammenden Messsignals mit einem Referenzsignal umfassen, das konventionell, z.B. über eine invasive Bestimmung erhalten worden ist. Dieser Abgleich kann im Rahmen der Erstbenutzung der erfindungsgemäßen Vorrichtung erfolgen und - sofern erforderlich - in zeitlichen Abständen, z.B. täglich, jeden zweiten Tag, wöchentlich etc., wiederholt werden. Vorzugsweise erfolgt der Abgleich durch Justierung von Auflageposition und/oder Auflagedruck für den zu untersuchenden Körperbereich, um ein stabiles reproduzierbares Messsignal zu erhalten, das dem Referenzsignal möglichst weitgehend entspricht. Hierzu kann die Vorrichtung Sensoren zur Erfassung und/oder Kontrolle der Auflageposition des zu bestrahlenden Körperbereichs, z.B. hinsichtlich der x-, y- und z-Koordinaten bezüglich des Auflageelements, und/oder zur Erfassung und/oder Kontrolle des Auflagedrucks, z.B. in einem Bereich von etwa 0,5-10 N, vorzugsweise im Bereich von etwa 1-5 N und besonders bevorzugt etwa 2 N enthalten. Ein Sensor zur Erfassung und/oder Kontrolle des Auflagedrucks kann z.B. eine Wägezelle enthalten. Ein Sensor zur Erfassung und/oder Kontrolle der Auflageposition kann eine Kamera, z.B. eine CCD-Kamera, einen Pulssensor und/oder einen Temperatursensor enthalten.

Die durch Adaption ermittelten Einstellungen für Auflageposition und/oder Auflagedruck werden vorzugsweise von der Vorrichtung registriert und gespeichert. Bei einer nachfolgenden Benutzung des Geräts können dann die korrekte Auflageposition bzw. der korrekte Auflagedruck durch ein Signal angezeigt werden, z.B. durch ein optisches und/oder aktustisches Signal. Die Messung des zu bestimmenden Analyten wird erst gestartet, wenn die vorgegebenen korrekten Einstellungen verifiziert worden sind.

Weiterhin enthält die Vorrichtung eine Einheit (b) zur Erfassung von körpereigener IR-Strahlung aus dem untersuchten Körperbereich, die zur separaten Erfassung von IR-Strahlung bei mindestens zwei unterschiedlichen Wellenlängen oder Wellenlängenbereichen in der Region von 0,7-20 µm, vorzugsweise von 3-20 µm, besonders bevorzugt von 8-12 µm, eingerichtet ist. In einer besonders bevorzugten Ausführungsform ist diese Einheit zur separaten Erfassung von IR-Strahlung bei mindestens zwei unterschiedlichen Wellenlängen oder Wellenlängenbereichen in der Region von 5-15 µm, insbesondere 7-12 µm und am meisten bevorzugt von 8-10 µm eingerichtet.

In einer Ausführungsform enthält die Erfassungseinheit (b) mehrere Sensoren, die zur separaten Erfassung von IR-Strahlung mit mindestens zwei unterschiedlichen Wellenlängen oder Wellenlängenbereichen vorgesehen sind. In einer anderen Ausführungsform kann die Vorrichtung wiederum einen Sensor enthalten, der zur zeitabhängigen separaten Erfassung von IR-Strahlung mit mindestens zwei unterschiedlichen Wellenlängen oder Wellenlängenbereichen vorgesehen ist.

Dabei ist die Erfassungseinheit (b) zur separaten Erfassung von IR-Strahlung bei mindestens einer ersten Wellenlänge bzw. einem ersten Wellenlängenbereich und bei mindestens einer zweiten Wellenlänge bzw. mindestens einem zweiten Wellenlängenbereich eingerichtet. Die erste Wellenlänge bzw. der erste Wellenlängenbereich liegt vorzugsweise in der Region eines Absorptionsminimums für den zu bestimmenden Analyten. Die zweite Wellenlänge bzw. der zweite Wellenlängenbereich liegt vorzugsweise in der Region eines Absorptionsbands des zu bestimmenden Analyten, d.h. in einer Region, in der der Analyt eine starke Absorption, vorzugsweise ein Absorptionsmaximum zeigt.

In einer Ausführungsform enthält die Vorrichtung mindestens einen ersten Sensor und mindestens einen zweiten Sensor, die jeweils zur separaten Erfassung von IR-Strahlung mit unterschiedlichen Wellenlängen bzw. Wellenlängenbereichen in der Region von 0,7-20 µm, vorzugsweise von 3-20 µm, besonders bevorzugt von 8-12 µm, eingerichtet sind. Ein erster Sensor wird dabei so ausgewählt, dass er zur Erfassung von IR-Strahlung mit einer ersten Wellenlänge bzw. mit einem ersten Wellenlängenbereich eingerichtet ist, wo die Intensität der körpereigenen IR-Strahlung von der Konzentration des zu bestimmenden Analyten im Wesentlichen unabhängig ist. Ein zweiter Sensor wird derart ausgewählt, dass er zur Erfassung von IR-Strahlung mit einer zweiten Wellenlänge bzw. einem zweiten Wellenlängenbereich eingerichtet ist, wo sich die Intensität der körpereigenen IR-Strahlung abhängig von der Konzentration des zu bestimmenden Analyten ändert. Dabei kann die erfindungsgemäße Vorrichtung jeweils einen oder mehrere erste und zweite Sensoren enthalten.

Der erste und/oder der zweite Sensor können als Wellenlängenunspezifische Strahlungssensoren, beispielsweise als Bolometer, ausgebildet sein, die mit optischen Filterelementen ausgestattet sind, die für eine jeweils zu erfassende Wellenlänge bzw. einen zu erfassenden Wellenlängenbereich durchlässig sind, um auf diese Weise eine Wellenlängen(bereichs)-spezifische Erfassung der IR-Strahlung zu ermöglichen. Hierzu können geeignete Filterelemente, z.B. Bandpass-, Hochpass- oder Tiefpassfilterelemente oder Kombinationen mehrerer solcher Filterelemente verwendet werden. In einer bevorzugten Ausführungsform werden als erster und/oder zweiter Sensor Bolometer mit hoher Präzision verwendet.

In einer Ausführungsform können zur Wellenlängen-spezifischen Erfassung von IR-Strahlung der erste und/oder der zweite Sensor mit engen Bandpassfilterelementen ausgestattet sein, die eine Durchlässigkeitsbreite von jeweils z.B. bis zu 0,8 µm, bis zu 0,4 µm, bis zu 0,3 µm oder bis zu 0,2 µm um die erste oder zweite Messwellenlänge aufweisen.

In noch einer weiteren Ausführungsform können die Filterelemente des ersten und/oder des zweiten Sensors Kombinationen von weiten Bandpassfilterelementen mit einer Durchlässigkeitsbreite von z.B. 2-12 µm, vorzugsweise 3-8 µm, Hochpassfilterelementen und/oder Tiefpassfilterelementen umfassen. So kann ein weites Bandpassfilterelement vorgesehen sein, das für IR-Strahlung in einer den gesamten Messwellenlängenbereich überdeckenden Region durchlässig ist, beispielsweise in einer Region von 8-10,5 µm oder 7-14 µm. Dieses Bandpassfilterelement kann in Kombination mit Tiefpass- und/oder Hochpassfilterelementen eingesetzt werden, um für den ersten und zweiten Sensor jeweils eine separate Erfassung von IR-Strahlung unterschiedlicher Wellenlängen oder Wellenlängenbereichen zu ermöglichen. Dabei kann einer der beiden Sensoren ein Tiefpassfilterelement enthalten, welches für IR-Strahlung bis hinab zu einer Grenzwellenlänge durchlässig ist, die zwischen der ersten Wellenlänge bzw. dem ersten Wellenlängenbereich und der zweiten Wellenlänge bzw. dem zweiten Wellenlängenbereich liegt. Alternativ oder zusätzlich kann der andere der beiden Sensoren ein Hochpassfilterelement enthalten, das für IR-Strahlung bis hinauf zu einer zweiten Grenzwellenlänge durchlässig ist, die ebenfalls zwischen der ersten Messwellenlänge bzw. dem ersten Messwellenlängenbereich und der zweiten Messwellenlänge bzw. dem zweiten Messwellenlängenbereich liegt.

Der Vorteil einer Verwendung von breiten Bandpassfiltern in Kombination mit einem Tiefpass- und/oder Hochpassfilter besteht darin, dass breitere Wellenlängenbereiche erfasst werden können. Auf diese Weise kann die Effizienz der Messung deutlich erhöht werden.

In einer konkreten Ausgestaltung dieser Ausführungsform kann einer der beiden Sensoren ein weites Bandpassfilter in Kombination mit einem Tiefpassfilter und der andere der beiden Sensoren ein breites Bandpassfilter in Kombination mit einem Hochpassfilter enthalten. In einer zweiten Ausgestaltung kann einer der beiden Sensoren ggf. nur ein breites Bandpassfilter und der andere der beiden Sensoren die Kombination eines Bandpassfilters mit einem Tiefpassfilter oder alternativ die Kombination eines Bandpassfilters mit einem Hochpassfilter enthalten. Bei den beiden letztgenannten Ausgestaltungsformen überlappen die von den beiden Sensoren erfassten Wellenlängenbereiche, so dass bei der Auswertung dieser überlappende Bereich herausgerechnet werden muss.

In noch einer weiteren Ausführungsform können der erste Sensor und/oder der zweite Sensor auch als Wellenlängen(bereichs)-spezifische Sensoren, beispielsweise als Quantenkaskaden-Sensoren ausgebildet sein.

In einer anderen Ausführungsform enthält die Erfassungseinheit (b) mindestens einen Sensor, der zur zeitabhängigen separaten Erfassung von IR-Strahlung mit mindestens zwei unterschiedlichen Wellenlängen bzw. Wellenlängenbereichen in der Region von 0,7-20 µm, vorzugsweise von 3-20 µm eingerichtet, besonders bevorzugt von 8-12 µm, ist, wobei bei einer ersten Wellenlänge bzw. einem ersten Wellenlängenbereich die Intensität der körpereigenen IR-Strahlung von der Konzentration des zu bestimmenden Analyten im Wesentlichen unabhängig ist, und
wobei bei einer zweiten Wellenlänge bzw. einem zweiten Wellenlängenbereich sich die Intensität der körpereigenen IR-Strahlung abhängig von der Konzentration des zu bestimmenden Analyten ändert.

Dabei kann der zur zeitabhängigen separaten Erfassung von Strahlung mit unterschiedlichen Wellenlängen oder Wellenlängenbereichen vorgesehene Sensor als Fabry-Perot Interferometer ausgebildet sein, beispielsweise als MEMS-Spektrometer für den MIR/TIR-Bereich von etwa 3-12 µm (siehe z.B. Tuohinieni et al., J. Micromech. Microeng. 22 (2012), 115004; Tuohinieni et al., J. Micromech. Microeng. 23 (2013), 075011).

Zusätzlich kann die Erfassungseinheit (c) gegebenenfalls noch mindestens einen weiteren Sensor enthalten, der zur unspezifischen Erfassung von körpereigener IR-Strahlung aus dem bestrahlten Körperbereich des Testsubjekts eingerichtet ist und zur Referenzierung, z.B. zur Referenzierung der Körpertemperatur dienen kann.

Die Größe der Sensoren der erfindungsgemäßen Vorrichtung kann je nach Bedarf gewählt werden. Beispielsweise können sie eine Querschnittsfläche im Bereich von 0,5-10 mm² aufweisen.

Die Sensoren der erfindungsgemäßen Vorrichtung befinden sich vorzugsweise in einem Zielstand der thermischen Äquilibrierung, z.B. indem sie in Kontakt mit einem gemeinsamen wärmeleitfähigen Träger wie etwa einem Körper, einer Platte oder einer Folie aus Metall, z.B. Kupfer oder Messing, stehen.

In einer bevorzugten Ausführungsform sind im Strahlengang zwischen dem zu untersuchenden Körperbereich und der Erfassungseinheit (b) optische Fokussierelemente, z.B. Linsenelemente, angeordnet, um eine möglichst punktförmige Fokussierung der körpereigenen IR-Strahlung auf dem Sensor bzw. den Sensoren der Erfassungseinheit (b) zu ermöglichen. So können der erste und/oder zweite Sensor mit optischen Fokussierelementen, z.B. Plankonvexlinsen oder Bikonvexlinsen, insbesondere Kugellinsen, aus IR-transparenten Materialien wie etwa Germanium oder Zinkselenid, ausgestattet sein, wobei der Linsendurchmesser günstigerweise dem Durchmesser des Sensors angepasst ist. Durch Verwendung einer optischen Anordnung mit fokussiertem Strahlengang kann die Strahlungsausbeute und somit die Sensitivität und Präzision der Messung gesteigert werden. Besonders bevorzugt ist die Verwendung von Sensoren, z.B. Bolometern, die mit Bikonvexlinsen, insbesondere Kugellinsen, ausgestattet sind.

Die erfindungsgemäße Vorrichtung kann zur Bestimmung von Analyten verwendet werden, die charakteristische Absorptionsbanden im IR-Bereich, insbesondere in den zuvor genannten Wellenlängenregionen, z.B. in den Regionen von 3-20 µm und insbesondere von 7-15 µm und am meisten bevorzugt von 8-12 µm aufweisen. Beispiele für solche Analyten sind Glucose oder andere klinische relevante Analyten wie etwa Lactat, Troponin oder C-reaktives Protein.

In einer besonders bevorzugten Ausführungsform ist die erfindungsgemäße Vorrichtung zur nicht-invasiven Bestimmung von Glucose in Blut, insbesondere in Kapillarblut der Dermis, eingerichtet. In diesem Fall umfasst die erste Wellenlänge bzw. der erste Wellenlängenbereich ein Absorptionsminimum von Glucose und die zweite Wellenlänge bzw. der zweite Wellenlängenbereich ein Absorptionsband von Glucose oder einen Teil davon. Beispielsweise kann die erste Wellenlänge in den Regionen von 8,1 ± 0,2 µm und/oder 8,5 ± 0,2 µm, insbesondere in den Regionen von 8,1 ± 0,1 µm und/oder 8,5 ± 0,1 µm liegen oder zumindest eine dieser Regionen umfassen. In diesen Wellenlängenregionen weist Glucose ein Absorptionsminimum auf. Die zweite Wellenlänge kann in der Region von 9,1 ± 0,2 µm, 9,3 ± 0,2 µm und/oder 9,6 ± 0,2 µm, insbesondere in der Region von 9,1 ± 0,1 µm, 9,3 ± 0,1 µm und/oder 9,6 ± 0,1 µm liegen oder zumindest eine dieser Regionen umfassen. In diesen Wellenlängenregionen weist Glucose ein Absorptionsband mit mehreren Absorptionsmaxima auf.

Ein weiterer Bestandteil der erfindungsgemäßen Vorrichtung ist eine Einheit zur Auswertung der von der Erfassungseinheit stammenden Signale und zur Ermittlung der Konzentration des Analyten auf Basis der ausgewerteten Signale. Die Auswertung der Signale beruht darauf, dass die körpereigene IR-Strahlung bei einer Wellenlänge oder einem Wellenlängenbereich aus einem Absorptionsminimum des Analyten, z.B. Glucose, unabhängig von der im Blut des Testsubjekts vorliegenden Analytkonzentration ist. Die körpereigene IR-Strahlung mit einer Wellenlänge oder einem Wellenlängenbereich aus einem Absorptionsband des Analyten ist hingegen wiederum von der Konzentration des betreffenden Analyten im Blut des Testsubjekts abhängig. Auf Basis des differenziellen Signals vom ersten und zweiten Sensor lässt sich eine ausreichend genaue Bestimmung der Analytkonzentration durchführen, insbesondere wenn eine selektive Auswertung von IR-Strahlung erfolgt, die aus Blutgefäßen, z.B. aus Blutgefäßen der Dermis und/oder Subkutis, vorzugsweise aus Kapillarblutgefäßen der Dermis stammt.

Noch ein weiterer Bestandteil der erfindungsgemäßen Vorrichtung ist eine Verkleidung bzw. ein Gehäuse, das eine thermische Isolierung gegenüber der Umgebung bewirkt. Geeignete Materialien für diesen Zweck sind thermisch nicht bzw. nur gering leitfähige Kunststoffe. Dabei kann die Verkleidung bzw. das Gehäuse so ausgestaltet sein, dass der in der Aufnahmeeinheit (a) eingebrachte, zu untersuchende Körperbereich zumindest im Wesentlichen von der Umgebung thermisch isoliert ist, z.B. indem eine dehnbare Hülle oder Membran aus thermisch nicht bzw. nur gering leitfähigem Material vorgesehen ist.

Die innere Oberfläche der Messvorrichtung kann außerdem mit einem für IR-Strahlung nicht reflektierenden und/oder einem IR-Strahlung absorbierenden Material vollständig oder teilweise beschichtet oder ausgestattet sein.

Ein weiterer Aspekt der Erfindung ist ein Verfahren zur nicht-invasiven quantitative Bestimmung eines Analyten im Blut eines Testsubjekts, umfassend die Schritte:
(i) separates Erfassen von körpereigener IR-Strahlung aus einem von dem Testsubjekt stammenden Körperbereich mit mindestens einer ersten Wellenlänge oder einem ersten Wellenlängenbereich in der Region von 0,7-20 µm, vorzugsweise von 3-20 µm, besonders bevorzugt von 8-12 µm, wo die Intensität der körpereigenen IR-Strahlung von der Konzentration des zu bestimmenden Analyten im Wesentlichen unabhängig ist, und mit mindestens einer zweiten Wellenlänge oder einem zweiten Wellenlängenbereich in der Region von 0,7-20 µm, vorzugsweise von 3-20 µm, besonders bevorzugt von 8-12 µm, wo sich die Intensität der körpereigenen IR-Strahlung abhängig von der Konzentration des zu bestimmenden Analyten ändert, und gegebenenfalls unspezifisches Erfassen von körpereigener IR-Strahlung aus dem Körperbereich zur Referenzierung,
(ii) Auswerten der in (i) erfassten Signale, wobei eine selektive Auswertung von körpereigener IR-Strahlung aus Blutgefäßen des Körperbereichs, insbesondere aus Kapillarblutgefäßen der Dermis erfolgt und
(iii) Ermitteln der Konzentration des Analyten auf Basis der ausgewerteten Signale.

Die Durchführung des Verfahrens erfolgt vorzugsweise ohne Verwendung einer externen Quelle für IR-Strahlung, z.B. unter Verwendung der zuvor beschriebenen Vorrichtung. Die im Rahmen der Vorrichtung spezifisch offenbarten Merkmale beziehen sich ebenso auf das Verfahren.

Die Vorrichtung und das Verfahren sind insbesondere zur Bestimmung von Glucose in Blut geeignet.

Weitere Aspekte der Erfindung sind wie folgt:
Vorrichtung zur nicht-invasiven quantitativen Bestimmung eines Analyten im Körper eines Testsubjekts, insbesondere in Blut, umfassend
   (a) eine Einheit zur Aufnahme des zu untersuchenden Körperbereichs, die ein für IR-Strahlung in der Wellenlängenregion von 0,7-20 µm, vorzugsweise von 3-20 µm, besonders bevorzugt von 8-12 µm, zumindest teilweise transparentes Element zur Auflage des zu untersuchenden Körperbereichs, das gegebenenfalls mit einem Temperaturstabilisierungselement, insbesondere einem Peltier-Element, versehen ist, und ein Element zur Erfassung und/oder Kontrolle der Auflageposition und/oder des Auflagedrucks für den zu untersuchenden Körperbereich umfasst,
   (b) eine Einheit zur Erfassung von körpereigener IR-Strahlung aus dem Körperbereich, die zur separaten Erfassung von IR-Strahlung bei mindestens zwei unterschiedlichen Wellenlängen oder Wellenlängenbereichen in der Region von 0,7-20 µm, vorzugsweise von 3-20 µm, besonders bevorzugt von 8-12 µm, eingerichtet ist,
      wobei bei einer ersten Wellenlänge oder einem ersten Wellenlängenbereich die Intensität der körpereigenen IR-Strahlung von der Konzentration des zu bestimmenden Analyten im Wesentlichen unabhängig ist, und
      wobei bei einer zweiten Wellenlänge oder einem zweiten Wellenlängenbereich sich die Intensität der körpereigenen IR-Strahlung abhängig von der Konzentration des zu bestimmenden Analyten ändert und
      wobei die Einheit gegebenenfalls zusätzlich zur nicht-selektiven Erfassung von körpereigener IR-Strahlung eingerichtet ist, und
   (c) eine Einheit, die zur Auswertung der von der Erfassungseinheit (b) stammenden Signale und zur Ermittlung der Konzentration des Analyten auf Basis der ausgewerteten Signale eingerichtet ist.

Vorrichtung zur nicht-invasiven quantitativen Bestimmung eines Analyten im Körper eines Testsubjekts, insbesondere in Blut, umfassend
(a) eine Einheit zur Aufnahme des zu untersuchenden Körperbereichs,
(b) eine Einheit zur Erfassung von körpereigener IR-Strahlung aus dem bestrahlten Körperbereich, die zur separaten Erfassung von IR-Strahlung bei mindestens zwei unterschiedlichen Wellenlängen oder Wellenlängenbereichen in der Region von 0,7-20 µm, vorzugsweise von 3-20 µm, besonders bevorzugt von 8-12 µm, eingerichtet ist,
   wobei bei einer ersten Wellenlänge oder einem ersten Wellenlängenbereich die Intensität der körpereigenen IR-Strahlung von der Konzentration des zu bestimmenden Analyten im Wesentlichen unabhängig ist, und
   wobei bei einer zweiten Wellenlänge oder einem zweiten Wellenlängenbereich sich die Intensität der körpereigenen IR-Strahlung abhängig von der Konzentration des zu bestimmenden Analyten ändert und
   wobei die Einheit gegebenenfalls zusätzlich zur nicht-selektiven Erfassung von reflektierter IR-Strahlung eingerichtet ist, und
   wobei die Einheit mindestens einen Sensor umfasst, der zur zeitabhängigen separaten Erfassung von körpereigener IR-Strahlung bei der ersten Wellenlänge oder dem ersten Wellenlängenbereich und bei der zweiten Wellenlänge oder einem zweiten Wellenlängenbereich eingerichtet ist, und
(c) eine Einheit, die zur Auswertung der von der Erfassungseinheit (b) stammenden Signale und zur Ermittlung der Konzentration des Analyten auf Basis der ausgewerteten Signale eingerichtet ist.

Vorrichtung zur nicht-invasiven quantitativen Bestimmung eines Analyten im Körper eines Testsubjekts, insbesondere in Blut, wie zuvor beschrieben, umfassend insbesondere Mittel zur Erfassung und/oder Kontrolle der Auflageposition und/oder des Auflagedrucks des zu bestrahlenden Körperbereichs, in Kombination mit einer Vorrichtung zur invasiven quantitativen Bestimmung des Analyten als Referenzierung des Messsignals bei der nicht-invasiven quantitativen Bestimmung des Analyten.

Die zuvor spezifisch offenbarten Merkmale beziehen sich ebenso auf die zuletzt genannten Vorrichtungen und auf Verwendung dieser Vorrichtungen basierende Verfahren. Diese Vorrichtungen und Verfahren sind beispielsweise zur Bestimmung von Glucose im Körper eines menschlichen Testsubjekts, insbesondere in Blut geeignet.

Im Folgenden wird die vorliegende Erfindung nochmals ausführlich näher erläutert. Die menschliche Haut besteht von außen nach innen aus mehreren Schichten und zwar der Oberhaut (Epidermis) mit der Hornschicht, verhornender Schicht und Keimschicht, der Lederhaut (Dermis) mit der Papillarschicht und der Netzschicht und der Unterhaut (Subkutis). Die Epidermis enthält keine Blutgefäße. Die Dermis enthält feine Kapillarblutgefäße, die mit größeren Blutgefäßen in der Subkutis in Verbindung stehen. Ein arterieller Puls liegt in dem mit Kapillargefäßen durchzogenen Bereich der Dermis, nicht jedoch in darüber liegenden Hautschichten, beispielsweise der Epidermis, vor. Von der Hautoberfläche ausgestrahlte IR-Strahlung, insbesondere IR-Strahlung in der Wellenlängenregion von 0,7-20 µm, vorzugsweise von 3-20 µm, besonders bevorzugt von 8-12 µm, stammt zumindest teilweise aus dem von Kapillargefäßen durchzogenen Bereich der Dermis. In diesem Bereich befindliche Substanzen mit Absorptionsbändern in der IR-Region können Strahlung im Bereich dieser Absorptionsbänder absorbieren, wobei das Ausmaß der Absorption mit der Konzentration der betreffenden Substanz korreliert. Die körpereigene IR-Strahlung stammt aus unterschiedlichen Regionen des untersuchten Körperbereichs, wobei eine aus der Epidermis stammende Strahlung keine Abhängigkeit vom arteriellen Puls des Testsubjekts aufweist. Im Gegensatz dazu weist körpereigene IR-Strahlung, die aus dem mit Kapillargefäßen durchzogenen Bereich der Dermis stammt, ein vom arteriellen Puls des Testsubjekts abhängiges Signal auf.

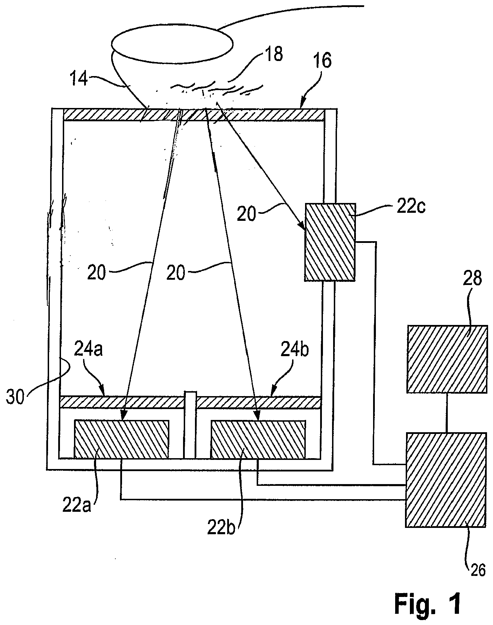

Die schematische Darstellung einer Ausführungsform der erfindungsgemäßen Vorrichtung ist in **Figur 1** gezeigt. In die Vorrichtung wird ein zu untersuchender Körperbereich (14) eines Testsubjekts eingebracht.

Dabei wird vorzugsweise ein gut durchbluteter Körperbereich, wie etwa eine Fingerkuppe, ausgewählt. Der Körperbereich (14) liegt auf einem Auflageelement (16), welches für die aus dem Körperbereich (14) stammende IR-Strahlung (20) zumindest teilweise, d.h. zumindest im Bereich der Messwellenlängen, optisch transparent ist. Das Auflageelement (16) kann Mittel, z.B. Sensoren, zur Erfassung und/oder Kontrolle der Auflageposition und/oder des Auflagedrucks des zu bestrahlenden Körperbereichs enthalten. Ferner kann das Auflageelement ein Temperaturstabilisierungselement enthalten.

IR-Strahlung (20) aus dem zu untersuchenden Körperbereich (14) stammt zumindest teilweise aus den oberflächennahen Blutkapillaren im Bereich der Dermis (18), die in einem Abstand von etwa 2,5 bis 3 mm zur Körperoberfläche liegen. Ein in den Blutkapillaren oder gegebenenfalls im angrenzenden Gewebe befindlicher Analyt wird die Strahlung im Bereich seines spezifischen Absorptionsbandes absorbieren, wobei das Ausmaß der Absorption mit der Konzentration des Analyten korreliert. Nicht absorbierte IR-Strahlung (20) tritt aus dem Körperbereich (14) aus.

Die Vorrichtung enthält weiterhin einen ersten Sensor (22a) und einen zweiten Sensor (22b) zur separaten Erfassung der körpereigenen IR-Strahlung bei unterschiedlichen Wellenlängen bzw. Wellenlängenbereichen (20) in der Region von 0,7-20 µm, vorzugsweise von 3-20 µm. Der erste Sensor (22a) ist zur selektiven Erfassung von körpereigener Strahlung mit einer ersten Wellenlänge oder einem ersten Wellenlängenbereich aus einem Absorptionsminimum des Analyten eingerichtet, wobei ein erstes Filterelement (24a) vorgesehen ist, welches für Strahlung mit der ersten Wellenlänge oder mit dem ersten Wellenlängenbereich selektiv durchlässig ist. Das heißt, das vom ersten Sensor gemessene Signal ist im Wesentlichen unabhängig von der Konzentration des zu bestimmenden Analyten. Der zweite Sensor (22b) ist wiederum zur selektiven Erfassung von körpereigener Strahlung (20) mit einer zweiten Wellenlänge oder einem zweiten Wellenlängenbereich aus einem Absorptionsband, vorzugsweise im Bereich eines Absorptionsmaximums des zu bestimmenden Analyten, eingerichtet, wobei ein zweites Filterelement (24b) vorgesehen ist, welches für Strahlung mit der zweiten Wellenlänge oder mit dem zweiten Wellenlängenbereich selektiv durchlässig ist. Dies bedeutet, dass das vom zweiten Sensor erfasste Signal abhängig von der Konzentration des Analyten ist.

Gegebenenfalls enthält die erfindungsgemäße Vorrichtung weiterhin einen dritten Sensor (22c), der zur unspezifischen Erfassung von körpereigener IR-Strahlung (20) eingerichtet ist und zur Referenzierung, z.B. zur Referenzierung der Körpertemperatur in dem zu untersuchenden Körperbereich (14) dient.

Die Sensoren (22a, 22b und gegebenenfalls 22c) können gegebenenfalls mit optischen Linsenelementen ausgestattet sein, z.B. Bikonvexlinsen, insbesondere Kugellinsen, um eine Fokussierung der auftreffenden körpereigenen IR-Strahlung zu ermöglichen.

Die von den Sensoren (22a, 22b und gegebenenfalls 22c) stammenden Signale werden an eine CPU-Einheit (26) zu deren Auswertung übermittelt. Auf Basis dieser Auswertung wird die Konzentration des Analyten bestimmt. Das Ergebnis kann dann in einem Display (28) angezeigt werden.

Die Innenseite (30) des Messsystems kann vollständig oder teilweise mit einer Oberfläche aus einem Material beschichtet oder ausgestattet sein, welche die von dem Körperbereich (14) stammende IR-Strahlung (20) nicht reflektiert und/oder die von dem Körperbereich (14) stammende IR-Strahlung (20) absorbiert.

Das Messsystem kann weiterhin eine Verkleidung oder ein Gehäuse aufweisen, das eine thermische Isolierung gegenüber der Umgebung bewirkt.

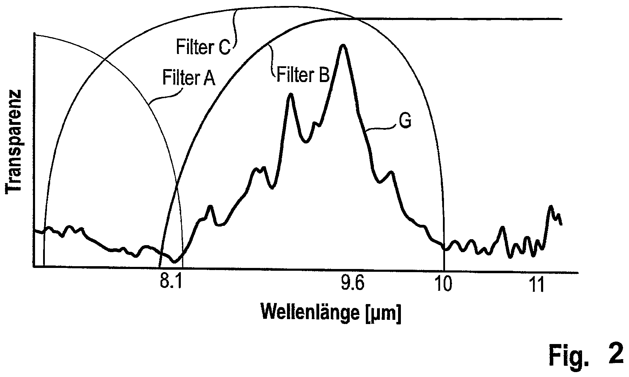

Eine alternative Ausführungsform zur separaten Erfassung von körpereigener IR-Strahlung bei unterschiedlichen Wellenlängen ist in **Figur 2** gezeigt. Die Absorptionskurve von Glucose in der Region zwischen 8 und 14 µm ist als fett gezeichnete Linie (G) dargestellt. Zur separaten Erfassung von IR-Strahlung mit zwei unterschiedlichen Wellenlängen aus dieser Region werden zwei Sensoren verwendet, die unterschiedliche Kombinationen von Bandpass-, Hochpass- und/oder Tiefpassfilterelementen umfassen. In einer Ausgestaltung enthalten beide Sensoren ein weites Bandpassfilter (C) mit einer Durchlässigkeit in der Region zwischen 8 und 14 µm. Beim ersten Sensor wird Filter (C) mit einem Hochpassfilter (A) kombiniert, der für IR-Strahlung mit einer Wellenlänge von etwa 8,5 µm oder weniger durchlässig ist. Ein Sensor, der mit den Filtern (A) und (C) ausgestattet ist, wird daher ein Signal aus der Region von 8-8,5 µm erfassen, das von der Konzentration der Glucose im Wesentlichen unabhängig ist. Der zweite Sensor ist mit einer Kombination des Bandpassfilters (C) mit einem Tiefpassfilter (B) ausgestattet, der für IR-Strahlung mit einer Wellenlänge von 8,5 µm oder höher durchlässig ist. Das mit diesem Sensor erfasste Signal umfasst ein in der Region von etwa 9-10 µm lokalisiertes Absorptionsband von Glucose und ist daher von der Glucosekonzentration abhängig. Durch differenzielle Auswertung der von beiden Sensoren erfassten Signale kann die Glucosekonzentration bestimmt werden.

In einer anderen Ausgestaltung kann auch ein erster Sensor mit dem Bandpassfilter (C) und dem Hochpassfilter (A) ausgestattet sein, während ein zweiter Sensor nur mit dem Bandpassfilter (C) ausgestattet ist. Das mit dem ersten Sensor erfasste Signal ist von der Glucosekonzentration unabhängig, während das vom zweiten Sensor erfasste Signal sich mit der Glucosekonzentration ändert.

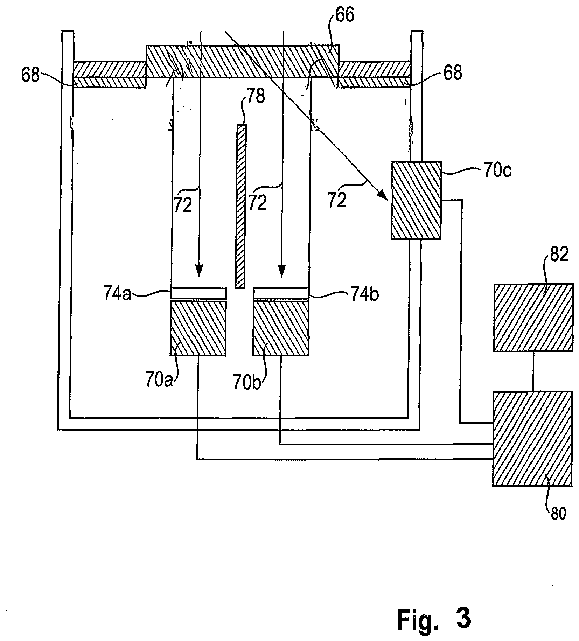

**Figur 3** zeigt die schematische Darstellung des Querschnitts einer weiteren Ausführungsform der erfindungsgemäßen Vorrichtung. Dabei ist der zu untersuchende Körperbereich des Testsubjekts (nicht gezeigt) auf einem Auflageelement (66) angeordnet sein, welches für IR-Strahlung zumindest teilweise optisch transparent ist. Beispielsweise handelt es sich bei dem Auflageelement um eine Si- oder Ge-Scheibe. Das Auflageelement (66) ist vorzugsweise mit einem Temperaturstabilisierungselement versehen, welches vorzugsweise ein Peltier-Element umfasst. Die Temperatur des Auflageelements kann dabei beispielsweise auf einen Bereich von etwa 1-31 °C, bevorzugt von etwa 25-30 °C, eingestellt werden. Vorzugsweise enthält das Auflageelement (60) Mittel, z.B. Sensoren (68), zur Erfassung und/oder Kontrolle des Auflagedrucks, wie etwa eine Wägezelle, sowie gegebenenfalls Sensoren zur Erfassung und/oder Kontrolle der Auflageposition, wie etwa eine Kamera, ein Pulssensor und/oder einen Temperatursensor. Günstigerweise wird ein Auflagedruck von etwa 1-5 N, z.B. etwa 2 N eingestellt.

Die Vorrichtung enthält weiterhin einen ersten Sensor (70a) und einen zweiten Sensor (70b) zur Erfassung der körpereigenen IR-Strahlung (72) mit unterschiedlichen Wellenlängen in der Region von 0,7-20 µm, vorzugsweise von 3-20 µm aus dem untersuchten Körperbereich. Der erste und der zweite Sensor können jeweils als Bolometer ausgebildet sein.

Der erste Sensor (70a) kann zur selektiven Erfassung von körpereigener IR-Strahlung mit einer ersten Wellenlänge aus einem Absorptionsminimum des Analyten eingerichtet sein, beispielsweise, wobei ein erstes Filterelement (74a), z.B. ein Bandpassfilter mit enger Durchlässigkeit vorgesehen ist, welches für Strahlung mit der ersten Wellenlänge selektiv durchlässig ist, bei der das Signal im Wesentlichen unabhängig von der Konzentration des zu bestimmenden Analyten ist. Für die Bestimmung von Glucose liegt die erste Wellenlänge vorzugsweise bei 8,1 ± 0,2 µm und/oder 8,5 ± 0,2 µm, besonders bevorzugt bei 8,1 ± 0,2 µm und/oder 8,5 ± 0,1 µm. Der zweite Sensor (74b) ist wiederum zur selektiven Erfassung von körpereigener IR-Strahlung (72) mit einer zweiten Wellenlänge aus einem Absorptionsband, vorzugsweise im Bereich eines Absorptionsmaximums des zu bestimmenden Analyten, eingerichtet, wobei ein zweites Filterelement (74b), z.B. ein Bandpassfilter mit enger Durchlässigkeit, vorgesehen ist, welches für Strahlung mit der zweiten Wellenlänge selektiv durchlässig ist. Bei der Bestimmung von Glucose liegt die zweite Wellenlänge vorzugsweise im Bereich von 9,1 ± 0,2 µm, 9,3 ± 0,2 µm und/oder 9,6 ± 0,2 µm, besonders bevorzugt im Bereich von 9,1 ± 0,1 µm, 9,3 ± 0,1 µm und/oder 9,6 ± 0,1 µm.

Alternativ kann anstelle von Bandpassfiltern mit enger Durchlässigkeit auch die in Figur 2 dargestellte Kombination eines Bandpassfilters mit weiter Durchlässigkeit sowie einem Hochpass- und/oder einem Tiefpassfilter verwendet werden.

Im Strahlengang der körpereigenen IR-Strahlung (72) zwischen dem zu untersuchenden Körperteil und den Sensoren (70a, 70b und gegebenenfalls 70c) können optische Fokussierelemente, z.B. Linsenelemente, angeordnet sein. So können die Sensoren (70a, 70b) gegebenenfalls mit optischen Linsenelementen ausgestattet sein, z.B. Bikonvexlinsen, insbesondere Kugellinsen, um eine Fokussierung der auftreffenden körpereigenen IR-Strahlung zu ermöglichen.

Gegebenenfalls enthält die erfindungsgemäße Vorrichtung weiterhin einen dritten Sensor (70c), der zur unspezifischen Erfassung von körpereigener IR-Strahlung (72) eingerichtet ist und zur Referenzierung, z.B. zur Referenzierung der Körpertemperatur des zu untersuchenden Körperbereichs dient. Der dritte Sensor (70c) kann als Bolometer ausgebildet sein.

Zwischen dem ersten Sensor (70a) und dem zweiten Sensor (70b) kann gegebenenfalls eine Zwischenwand (78) angeordnet sein.

Die von den Sensoren (70a, 70b und gegebenenfalls 70c) stammenden Signale werden an eine CPU-Einheit (80) zur Auswertung der Signale übermittelt. Auf Basis dieser Auswertung wird die Konzentration des Analyten bestimmt, das Ergebnis kann dann in einem Display (82) gezeigt werden. Die CPU-Einheit kann außerdem zur Steuerung der Anregungsquellen (60a, 60b) und/oder der Kühlelemente (64, 68) verwendet werden.

Vorzugsweise befinden sich die Sensoren (70a, 70b und gegebenenfalls 70c) in thermischer Äquilibrierung, indem sie in Kontakt mit thermisch leitfähigem Material, z.B. einem Körper, einer Platte oder einer Folie aus Metall, stehen.

Gegebenenfalls kann die innere Oberfläche des Messsystems mit einem für IR-Strahlung nicht reflektierenden und/oder einem IR-Strahlung absorbierenden Material beschichtet sein. Weiterhin kann das Messsystem eine thermische Isolierung gegenüber der Umgebung aufweisen.

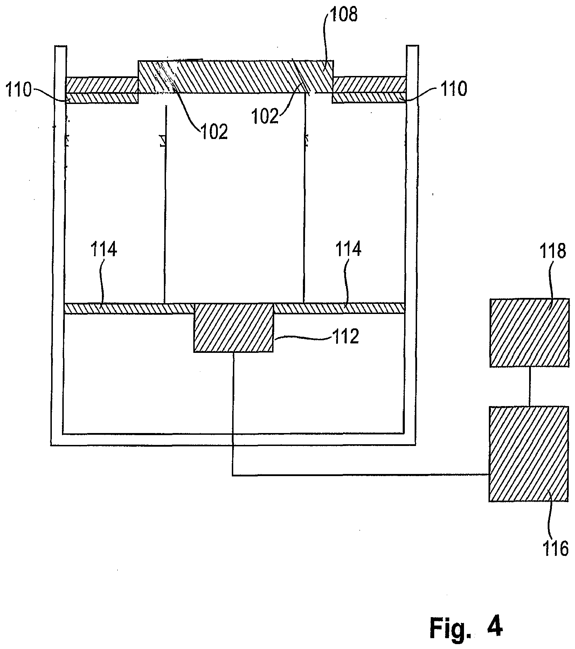

Die schematische Darstellung noch einer weiteren Ausführungsform der erfindungsgemäßen Vorrichtung im Querschnitt ist in **Figur 4** gezeigt.

Ein zu untersuchender Körperbereich des Testsubjekts (nicht gezeigt) ist auf einem Auflageelement (108) angeordnet, welches für die körpereigene IR-Strahlung (102) im Wesentlichen optisch transparent ist. Bevorzugte Ausführungsformen des Auflageelements sind wie zuvor beschrieben. Das Auflageelement (108) kann in Verbindung mit einem Temperaturstabilisierungselement stehen, das vorzugsweise ein Peltier-Element umfasst. Vorzugsweise enthält das Auflageelement Sensoren (110) zur Erfassung und/oder Kontrolle des Auflagedrucks des zu untersuchenden Körperbereichs und gegebenenfalls Sensoren zur Erfassung und/oder Kontrolle der Auflageposition des zu untersuchenden Körperbereichs.

Die Vorrichtung enthält weiterhin einen Sensor (112), der zeitabhängig eine Messung bei zwei unterschiedlichen Messwellenlängen ermöglicht. Vorzugsweise ist dieser Sensor als IR-MEMS-Sensor ausgebildet, der vorzugsweise IR-Strahlung im Bereich von 8-14 µm erfassen kann. Der Sensor (112) kann in Verbindung mit einem weiteren Temperaturstabilisierungselement (114) stehen, welches beispielsweise ein Peltier-Element umfassen kann. Der Sensor (112) kann gegebenenfalls mit einem Linsenelement, wie zuvor beschrieben, ausgestattet sein.

Die vom Sensor (112) stammenden Signale werden vorzugsweise über einen A/D-Konverter an eine CPU-Einheit (116) zur Auswertung der Signale übermittelt. Auf Basis dieser Auswertung wird die Konzentration des Analyten bestimmt. Das Ergebnis kann dann in einem Display (118) gezeigt werden. Die CPU-Einheit (116) kann weiterhin eingerichtet sein, um die Anregungsquellen (100a, 100b) und/oder die Temperaturstabilisierungselemente (106, 110, 114) zu steuern.

Die Innenoberfläche des Messsystems kann gegebenenfalls mit einer für IR-Strahlung nicht reflektierenden und/oder IR-Strahlung absorbierenden Beschichtung versehen sein. Weiterhin kann das Messsystem eine thermische Isolierung gegenüber der Umgebung aufweisen.

## Patentansprüche

1. Vorrichtung zur nicht-invasiven quantitativen Bestimmung eines Analyten im Blut eines Testsubjekts, umfassend:
(a) eine Einheit zur Aufnahme eines von dem Testsubjekt stammenden und zu untersuchenden Körperbereichs,
(b) eine Einheit zur Erfassung von körpereigener IR-Strahlung aus dem bestrahlten Körperbereich, die zur separaten Erfassung von IR-Strahlung bei mindestens zwei unterschiedlichen Wellenlängen oder Wellenlängenbereichen in der Region von 3-20 µm, vorzugsweise von 8-12 µm eingerichtet ist,
wobei bei einer ersten Wellenlänge oder einem ersten Wellenlängenbereich die Intensität der körpereigenen IR-Strahlung von der Konzentration des zu bestimmenden Analyten im Wesentlichen unabhängig ist, und
wobei bei einer zweiten Wellenlänge oder einem zweiten Wellenlängenbereich sich die Intensität der körpereigenen IR-Strahlung abhängig von der Konzentration des zu bestimmenden Analyten ändert und
wobei die Einheit gegebenenfalls zusätzlich zur unspezifischen Erfassung von körpereigener IR-Strahlung eingerichtet ist, und
(c) eine Einheit, die zur Auswertung der von der Erfassungseinheit (b) stammenden Signale und zur Ermittlung der Konzentration des Analyten auf Basis der ausgewerteten Signale eingerichtet ist, wobei die Einheit zur selektiven Auswertung von körpereigener IR-Strahlung eingerichtet ist, die aus Kapillarblutgefäßen der Dermis des bestrahlten Körperbereichs stammt.

2. Vorrichtung nach Anspruch 1,
**dadurch gekennzeichnet,**
**dass** sie keine externe Quelle zur Erzeugung von IR-Strahlung enthält.

3. Vorrichtung nach einem der Ansprüche 1 bis 2,
**dadurch gekennzeichnet,**
**dass** die Einheit zur Aufnahme des zu untersuchenden Körperoberflächenbereichs (a) ein für IR-Strahlung in der Wellenlängenregion von 3-20 µm, vorzugsweise von 8-12 µm zumindest teilweise transparentes Element zur Auflage des zu untersuchenden Körperbereichs umfasst, das gegebenenfalls mit einem Temperaturstabilisierungselement, insbesondere einem Peltier-Element, in Verbindung steht.

4. Vorrichtung nach einem der Ansprüche 1 bis 3,
**dadurch gekennzeichnet,**
**dass** die Einheit zur Aufnahme des zu untersuchenden Körperflächenbereichs (a) Mittel, z.B. Sensoren, zur Erfassung und/oder Kontrolle von Auflageposition und/oder Auflagedruck des zu untersuchenden Körperbereichs umfasst.

5. Vorrichtung nach Anspruch 4,
**dadurch gekennzeichnet,**
**dass** Mittel zur Erfassung und/oder Kontrolle eines Auflagedrucks im Bereich von etwa 0,5-10 N, vorzugsweise im Bereich von etwa 1-5 N und besonders bevorzugt von etwa 2 N vorgesehen sind.

6. Vorrichtung nach einem der Ansprüche 1 bis 5,
**dadurch gekennzeichnet**
**dass** die Einheit zur Erfassung von körpereigener IR-Strahlung (b) mindestens einen ersten Sensor, mindestens einen zweiten Sensor und gegebenenfalls mindestens einen dritten Sensor umfasst, wobei der erste Sensor zur Erfassung von IR-Strahlung mit einer ersten Wellenlänge oder einem ersten Wellenlängenbereich in der Region von 3-20 µm, vorzugsweise von 8-12 µm eingerichtet ist, wo die Intensität der körpereigenen IR-Strahlung von der Konzentration des zu bestimmenden Analyten im Wesentlichen unabhängig ist, wobei der zweite Sensor zur Erfassung von IR-Strahlung mit einer zweiten Wellenlänge oder einem zweiten Wellenlängenbereich in der Region von 3-20 mm, vorzugsweise von 8-12 µm eingerichtet ist, wo sich die Intensität der körpereigenen IR-Strahlung abhängig von der Konzentration des zu bestimmenden Analyten ändert, und wobei der dritte Sensor zur Referenzierung der körpereigenen IR-Strahlung eingerichtet ist.

7. Vorrichtung nach Anspruch 6,
**dadurch gekennzeichnet,**
**dass** der erste Sensor und/oder der zweite Sensor als Bolometer mit einem für die zu erfassende Wellenlänge oder den zu erfassenden Wellenlängenbereich durchlässigen Filterelement oder einer Kombination von Filterelementen oder als Quantenkaskaden-Sensoren ausgebildet sind.

8. Vorrichtung nach einem der Ansprüche 1 bis 7,
**dadurch gekennzeichnet,**
**dass** die Einheit zur Erfassung von körpereigener IR-Strahlung (b) mindestens einen Sensor umfasst, der zur zeitabhängigen separaten Erfassung von IR-Strahlung mit mindestens zwei unterschiedlichen Wellenlängen oder Wellenlängenbereichen in der Region von 3-20 µm, vorzugsweise von 8-12 µm, eingerichtet ist,
wobei bei einer ersten Wellenlänge oder einem ersten Wellenlängenbereich die Intensität der körpereigenen IR-Strahlung von der Konzentration des zu bestimmenden Analyten im Wesentlichen unabhängig ist, und
wobei bei einer zweiten Wellenlänge oder einem zweiten Wellenlängenbereich sich die Intensität der körpereigenen IR-Strahlung abhängig von der Konzentration des zu bestimmenden Analyten ändert, wobei der Sensor insbesondere als Fabry-Perot Interferometer ausgebildet ist.

9. Vorrichtung nach einem der Ansprüche 1 bis 8,
**dadurch gekennzeichnet,**
**dass** im Strahlengang zwischen dem zu untersuchenden Körperbereich und der Einheit zur Erfassung von körpereigener IR-Strahlung (b) ein optisches Fokussierelement angeordnet ist.

10. Vorrichtung nach einem der Ansprüche 1 bis 9,
**dadurch gekennzeichnet,**
**dass** die Einheit zur Erfassung von körpereigener IR-Strahlung (b) mindestens einen Sensor aufweist, der mit einem Linsenelement, insbesondere einer Bikonvexlinse, z.B. einer Kugellinse, ausgestattet ist.

11. Vorrichtung nach einem der Ansprüche 1 bis 10 zur Bestimmung von Glucose in Blut, wobei die erste Wellenlänge oder der erste Wellenlängenbereich ein Absorptionsminimum von Glucose und die zweite Wellenlänge oder der zweite Wellenlängenbereich ein Absorptionsband von Glucose oder einen Teil davon umfasst.

12. Vorrichtung nach Anspruch 11,
**dadurch gekennzeichnet,**
**dass** die erste Wellenlänge oder der erste Wellenlängenbereich die Regionen von 8,1 ± 0,2 µm und/oder 8,5 ± 0,2 µm, insbesondere die Regionen von 8,1 ± 0,1 µm und/oder 8,5 ± 0,1 µm umfasst und/oder die zweite Wellenlänge bzw. der zweite Wellenlängenbereich die Regionen von 9,1 ± 0,2 µm, 9,3 ± 0,2 µm und/oder 9,6 ± 0,2 µm, insbesondere die Region von 9,1 ± 0,1 µm, 9,3 ± 0,1 µm und/oder 9,6 ± 0,1 µm umfasst.

13. Verwendung einer Vorrichtung nach einem der Ansprüche 1 bis 12 zur nicht-invasiven quantitativen Bestimmung eines Analyten, insbesondere zur Bestimmung von Glucose im Blut eines Testsubjekts.

14. Verfahren zur nicht-invasiven quantitativen Bestimmung eines Analyten im Blut eines Testsubjekts, umfassend die Schritte:
(i) separates Erfassen von körpereigener IR-Strahlung aus einem von dem Testsubjekt stammenden Körperbereich mit mindestens einer ersten Wellenlänge oder einem ersten Wellenlängenbereich in der Region von 3-20 µm, vorzugsweise von 8-12 µm, wo die Intensität der körpereigenen IR-Strahlung von der Konzentration des zu bestimmenden Analyten im Wesentlichen unabhängig ist, und mit mindestens einer zweiten Wellenlänge oder einem zweiten Wellenlängenbereich in der Region von 3-20 µm, vorzugsweise von 8-12 µm, wo sich die Intensität der körpereigenen IR-Strahlung abhängig von der Konzentration des zu bestimmenden Analyten ändert, und gegebenenfalls unspezifisches Erfassen von körpereigener IR-Strahlung aus dem bestrahlten Körperoberflächenbereich zur Referenzierung,
(ii) Auswerten der in (i) erfassten Signale, wobei eine selektive Auswertung von reflektierter IR-Strahlung aus Kapillarblutgefäßen der Dermis des bestrahlten Körperbereichs erfolgt und
(iii) Ermitteln der Konzentration des Analyten auf Basis der ausgewerteten Signale.

15. Verfahren nach Anspruch 14,
**dadurch gekennzeichnet,**
**dass** das aus Blutgefäßen stammende Signal aufgrund seiner von der Pulsfrequenz des Testsubjekts abhängigen zeitlichen Variation bestimmt wird.
